# EUROPEAN PATENT SPECIFICATION

(11) **EP 2 517 135 B1**
(45) Date of publication and mention of the grant of the patent: **09.10.2019**
(21) Application number: 10813123.6
(22) Date of filing: 20.12.2010
(51) Int. Cl.: G16H 50/20, G16H 70/20, G16H 20/00

(54) **MAPPING PATIENT DATA INTO A MEDICAL GUIDELINE**
ABBILDEN VON PATIENTENDATEN AUF EINE MEDIZINSCHE RICHTLINIE
PROJECTION DES DONNÉES D'UN PATIENT SUR UNE LIGNE DIRECTRICE MÉDICALE

(30) Priority: 22.12.2009 US 288879 P
(43) Date of publication of application: 31.10.2012
(73) Proprietor: Koninklijke Philips N.V., 5656 AE Eindhoven (NL); Philips Intellectual Property & Standards GmbH, 20099 Hamburg (DE)
(72) Inventor: OPFER, Roland, NL-5656 AE Eindhoven (NL); TULIPANO, Paola, Karina, NL-5656 AE Eindhoven (NL); COHEN-SOLAL, Eric, NL-5656 AE Eindhoven (NL); VLOEMANS, Victor, Paulus, Marcellus, NL-5656 AE Eindhoven (NL); CARLSEN, Ingwer, Curt, NL-5656 AE Eindhoven (NL); VERBEEK, Alexander, Adrianus, Martinus, NL-5656 AE Eindhoven (NL); NICOLAAS, Arvid, Randal, NL-5656 AE Eindhoven (NL); DRIES, Sebastian, Peter, Michael, NL-5656 AE Eindhoven (NL); VAN OMMERING, Robbert, Christiaan, NL-5656 AE Eindhoven (NL)
(74) Representative: van Velzen, Maaike Mathilde
(86) International application number: PCT/IB2010/055946
(87) International publication number: WO 2011/077353

(56) References cited:
- WO-A1-2009/136354
- WO-A2-2005/029252
- WO-A2-2005/059803
- US-A1- 2006 116 908
- ISERN D ET AL: "Computer-based execution of clinical guidelines: A review", INTERNATIONAL JOURNAL OF MEDICAL INFORMATICS, ELSEVIER SCIENTIFIC PUBLISHERS, SHANNON, IR, vol. 77, no. 12, 1 December 2008 (2008-12-01), pages 787-808, XP025584756, ISSN: 1386-5056, DOI: DOI:10.1016/J.IJMEDINF.2008.05.010 [retrieved on 2008-07-17]
- TU S W ET AL: "The SAGE Guideline Model: Achievements and Overview", JOURNAL OF THE AMERICAN MEDICAL INFORMATICS ASSOCIATION (JAMIA), HANLEY AND BELFUS, PHILADELPHIA, PA, US, vol. 14, no. 5, 1 September 2007 (2007-09-01), pages 589-598, XP025536328, ISSN: 1067-5027, DOI: DOI:10.1197/JAMIA.M2399 [retrieved on 2007-09-01]

## Description

### FIELD OF THE INVENTION:

The invention relates to mapping a patient data structure for describing a patient's case into a guideline data structure for describing a medical guideline.

### BACKGROUND OF THE INVENTION

Guideline-based clinical decision support systems provide tools for the management of patient diseases and treatments. Due to further personalization of treatment, the complexity of clinical guidelines is increasing continuously. For the physician it is increasingly challenging to keep abreast of the latest state of the art for treatment and diagnosis recommendations. At the same time, due to enormous cost pressure on the healthcare system, evidence based medicine practice and guideline adherence is gaining importance. Therefore, systems which provide guideline-based clinical decision support are of high relevance.

Guideline based decision support systems often turn out to be too inflexible in practice. These systems often impose certain actions on the user, which do not always reflect clinical reality. For example, in some guideline implementations the user needs to click through a decision tree step by step. This is tedious and the order of nodes in the tree might not necessarily match with the actions taken by a physician. In addition, the guidelines may require performing a diagnostic test using equipment which is not available at the moment. While rescheduling some actions imposed by the guidelines is often possible, it may be difficult to have these actions, performed out of order, driven by a guideline.

US 2006/0116908 A1 discloses an apparatus and method for generating a patient's medical record. The apparatus comprises a data input component that executes through a plurality of clinical, tree-like pathways that are traversed as data describing the patient's condition is entered, for example, by the physician during a clinical examination. At each node, the physician is prompted as to the additional health information required to traverse the clinical pathway. Once an end "leaf' is reached, the medical record is generated based on the path traversed through the clinical pathway. A web site stores the record for access via a web browser.

### SUMMARY OF THE INVENTION

It would be advantageous to have a guideline driven system, capable of decoupling the data entry from the guideline functionality and thus allowing actions required or recommended by the guideline to be performed out of order. The guideline may stay in the background unless the user wants to obtain information as to whether the current treatment is compliant with evidence based care.

Thus, in an aspect, the invention provides a system for mapping a patient data structure for describing a patient's case into a guideline data structure for describing a medical guideline as defined in claim 1, the system comprising storage for storing:
- a plurality of data items;
- the patient data structure comprising data items of the plurality of data items;
- the guideline data structure comprising a guideline graph, wherein the guideline graph is a directed graph, the guideline graph comprising action nodes, wherein each action node is associated with an action;
- the system further comprising a linker for linking data items comprised in the patient data structure to action nodes of the guideline graph, based on a relation between said data items and actions associated with said action nodes, thereby mapping the patient data structure into the guideline data structure.

A typical action associated with each node is to perform a measurement on and/or apply a treatment to the patient. The data item related to performing the measurement comprises a measurement outcome and the data item related to applying a treatment comprises values of parameters of the applied treatment. By decoupling the data input functionality and the medical guideline functionality, the application of the method described in this invention results in fewer constraints on how to use the system. The guideline becomes an advisory tool rather than a tool determining the workflow of the physician. The system can positively influence the quality and completeness of the available patient data. Advantageously, mapping the patient data structure into the guideline graph of the guideline data structure provides an easy way of implementing and visualizing a personalized medical guideline which coincides with the general guideline requirements.

In an embodiment of the system, the guideline graph further comprises decision nodes, and each decision node is adapted for testing data items linked to action nodes connected directly or indirectly to said decision node. The results of these tests describe branching rules defined in branching points of the guideline graph. Decision nodes are useful nodes of every guideline graph.

In an embodiment, the system further comprises a display for displaying at least part of the guideline graph, wherein said at least part of the guideline graph comprises at least one action node to which data items comprised in the patient data structure are linked, and wherein said at least one action node is indicated in the displayed part of the guideline graph. Thus, a personalized medical guideline can be viewed by a user of the system.

In an embodiment of the system, the guideline graph further comprises a completeness estimator for computing a degree of completeness of an action comprised in an action node. Thus, the user can learn in a user-friendly way which actions need to be performed to satisfy the guideline requirements. In an embodiment, the guideline graph further comprises a completeness node for indicating the computed degree of completeness.

The system further comprises a pathway finder for computing a pathway of the patient in the guideline graph, the pathway comprising the at least one action node to which data items comprised in the patient data structure are linked. The pathway offers a clear overview of the personalized medical guideline showing actions which have been already completed and which need to be completed. Optionally, a degree of completeness of an action and other useful information may be displayed for each incomplete action. Advantageously, the pathway may comprise action nodes corresponding to actions with null degree of completion, which are not linked to any data item. Nevertheless, these nodes may be associated with actions relevant to the personalized medical guideline.

In an embodiment, the system further comprises a predictor for selecting, on the basis of the pathway and the guideline graph, at least one action node of the guideline graph which is not yet comprised in the pathway, and for including the selected at least one action node in the pathway. The selected action node defines a future action associated with the action node to be executed. The future node may be indicated in the displayed part of the guideline graph. In this way, the system is adapted for assisting a user in deciding on the tests to be performed on or the treatment to be applied to the patient. The data gathered by carrying out the tests, measurements, and/or treatment may comprise the test or measurement outcomes and values of parameters and/or outcomes of the applied treatment. This data can be entered into the storage as data items comprised in the patient data structure.

In an embodiment of the system, the guideline graph is a tree. Many guideline graphs may be implemented as decision trees. The current invention is also suitable for such relatively simple and common guideline graph implementations.

In a further aspect, the system according to the invention is comprised in a decision support system.

In a further aspect, the system according to the invention is comprised in a workstation.

In a further aspect, the invention provides a computer-implemented method of mapping a patient data structure for describing a patient's case into a guideline data structure for describing a medical guideline as defined in claim 11, wherein
- the patient data structure comprises data items of a plurality of data items;
- the guideline data structure comprises a guideline graph, wherein the guideline graph is a directed graph, the guideline graph comprising action nodes, wherein each action node is associated with an action;
- the method comprising linking data items comprised in the patient data structure to action nodes of the guideline graph, based on a relation between said data items and actions associated with said action nodes, thereby mapping the patient data structure into the guideline data structure.

In an implementation of the method, the guideline graph further comprises decision nodes, and each decision node is adapted for testing data items linked to action nodes connected directly or indirectly to said decision node.

In an implementation, the method further comprises at least one of the following steps:
- displaying at least part of the guideline graph, wherein said at least part of the guideline graph comprises at least one action node to which data items comprised in the patient data structure are linked, and wherein said at least one action node is indicated in the displayed part of the guideline graph;
- computing a degree of completeness of an action comprised in an action node; or
- selecting, on the basis of the pathway and the guideline graph, at least one action node of the guideline graph which is not yet comprised in the pathway, and including the selected at least one action node in the pathway.

In a further aspect, the invention provides a computer program product to be loaded by a computer arrangement, comprising instructions for mapping a patient data structure for describing a patient's case into a guideline data structure for describing a medical guideline, the computer arrangement comprising a processing unit and a memory, the computer program product, after being loaded, providing said processing unit with the capability to carry out steps of the method according to the invention.

It will be appreciated by those skilled in the art that two or more of the above-mentioned embodiments, implementations, and/or aspects of the invention may be combined in any way deemed useful.

Modifications and variations of the system, of the decision support system, of the workstation, of the method, and/or of the computer program product, which correspond to the described modifications and variations of the system or of the method, can be carried out by a person skilled in the art on the basis of the description.

The invention is defined in the independent claims. Advantageous embodiments are defined in the dependent claims.

### BRIEF DESCRIPTION OF THE DRAWINGS

These and other aspects of the invention are apparent from and will be elucidated by means of implementations and embodiments described hereinafter and with reference to the accompanying drawings, wherein:
Fig. 1 shows a block diagram of an exemplary embodiment of the system;
Fig. 2 illustrates the patient data structure comprising data items and a form for entering the data items;
Fig. 3 illustrates the guideline data structure and the guideline graph;
Fig. 4 schematically illustrates data items comprised in the data storage, linked to action nodes comprised in the guideline graph, and the decision node;
Fig. 5 shows an exemplary completeness node;
Fig. 6 shows an exemplary pathway in the guideline graph comprised in the guideline data structure;
Fig. 7 shows a guideline, wherein a care phase is assigned to each node;
Fig. 8A illustrates how to visualize care phase completeness on a list of patient summaries;
Fig. 8B illustrates how to visualize care phase completeness on a patient data form;
Fig. 9 schematically shows an exemplary flowchart of the method;
Fig. 10 schematically shows an exemplary embodiment of the decision support system; and
Fig. 11 schematically shows an exemplary embodiment of the workstation.
Identical reference numerals are used to denote similar parts throughout the Figures.

### DETAILED DESCRIPTION OF EMBODIMENTS

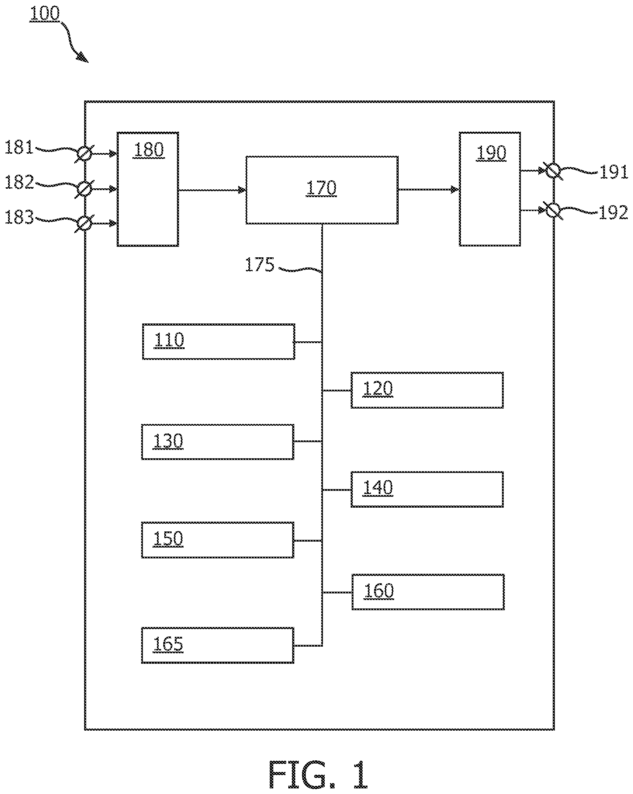

Fig. 1 schematically shows a block diagram of an exemplary embodiment of the system 100 for mapping a patient data structure for describing a patient's case into a guideline data structure for describing a medical guideline, the system comprising storage 170 for storing:
- a plurality of data items;
- the patient data structure comprising data items of the plurality of data items;
- the guideline data structure comprising a guideline graph, wherein the guideline data structure comprises a guideline graph, wherein the guideline graph is a directed graph, the guideline graph comprising action nodes, wherein each action node is associated with an action;
- the system further comprising a linker 110 for linking data items comprised in the patient data structure to action nodes of the guideline graph, based on a relation between said data items and actions associated with said action nodes, thereby mapping the patient data structure into the guideline data structure.

The exemplary embodiment of the system 100 further comprises:
- a display 120 for displaying at least part of the guideline graph, wherein said at least part of the guideline graph comprises at least one action node to which data items comprised in the patient data structure are linked, and wherein said at least one action node is indicated in the displayed part of the guideline graph;
- a completeness estimator 130 for computing a degree of completeness of an action comprised in an action node;
- a pathway finder 140 for computing a pathway of the patient in the guideline graph, the pathway comprising the at least one action node to which data items comprised in the patient data structure are linked;
- a predictor 150 for selecting, on the basis of the pathway and the guideline graph, at least one action node of the guideline graph which is not yet comprised in the pathway, and for including the selected at least one action node in the pathway;
- a control unit 160 for controlling the work of the system 100;
- a user interface 165 for communication between the user and the system 100; and
- a memory unit 170 for storing data.

In an embodiment of the system 100, there are three input connectors 181, 182 and 183 for the incoming data. The first input connector 181 is arranged to receive data coming in from a data storage means such as, but not limited to, a hard disk, a magnetic tape, a flash memory, or an optical disk. The second input connector 182 is arranged to receive data coming in from a user input device such as, but not limited to, a mouse or a touch screen. The third input connector 183 is arranged to receive data coming in from a user input device such as a keyboard. The input connectors 181, 182 and 183 are connected to an input control unit 180.

In an embodiment of the system 100, there are two output connectors 191 and 192 for the outgoing data. The first output connector 191 is arranged to output the data to a data storage means such as a hard disk, a magnetic tape, a flash memory, or an optical disk. The second output connector 192 is arranged to output the data to a display device. The output connectors 191 and 192 receive the respective data via an output control unit 190.

A person skilled in the art will understand that there are many ways to connect input devices to the input connectors 181, 182 and 183 and the output devices to the output connectors 191 and 192 of the system 100. These ways comprise, but are not limited to, a wired and a wireless connection, a digital network such as, but not limited to, a Local Area Network (LAN) and a Wide Area Network (WAN), the Internet, a digital telephone network, and an analog telephone network.

In an embodiment of the system 100, the system 100 comprises a memory unit 170. The system 100 is arranged to receive input data from external devices via any of the input connectors 181, 182, and 183 and to store the received input data in the memory unit 170. Loading the input data into the memory unit 170 allows quick access to relevant data portions by the units of the system 100. The input data comprises data items comprised in patient data structures. The memory unit 170 may be implemented by devices such as, but not limited to, a register file of a CPU, a cache memory, a Random Access Memory (RAM) chip, a Read Only Memory (ROM) chip, and/or a hard disk drive and a hard disk. The memory unit 170 may be further arranged to store the output data. The output data comprises a sub-graph of the guideline graph comprising the pathway, i.e. a patient's personalized medical guideline. The memory unit 170 may be also arranged to receive data from and/or deliver data to the units of the system 100 comprising the linker 110, the display 120, the completeness estimator 130, the pathway finder 140, the predictor 150, the control unit 160, and the user interface 165, via a memory bus 175. The memory unit 170 is further arranged to make the output data available to external devices via any of the output connectors 191 and 192. Storing data from the units of the system 100 in the memory unit 170 may advantageously improve performance of the units of the system 100 as well as the rate of transfer of the output data from the units of the system 100 to external devices.

In an embodiment of the system 100, the system 100 comprises a control unit 160 for controlling the system 100. The control unit 160 may be arranged to receive control data from and provide control data to the units of the system 100. For example, after computing the pathway, the pathway finder 140 may be arranged to provide control data "the pathway is computed" to the control unit 160, and the control unit 160 may be arranged to provide control data "select new action nodes" to the predictor 150. Alternatively, a control function may be implemented in another unit of the system 100. For example, control functions may be distributed among the remaining units of the system 100.

In an embodiment of the system 100, the system 100 comprises a user interface 165 for communication between a user and the system 100. The user interface 165 may be arranged to receive a user input comprising data items for patient data structures. Optionally, the user interface may receive a user input for selecting a mode of operation of the system such as, e.g., for selecting action nodes of the guideline graph which are not yet comprised in the pathway, based on the computed pathway and the guideline graph. The user interface may be further arranged to display the computed pathway. A person skilled in the art will understand that more functions may be advantageously implemented in the user interface 165 of the system 100.

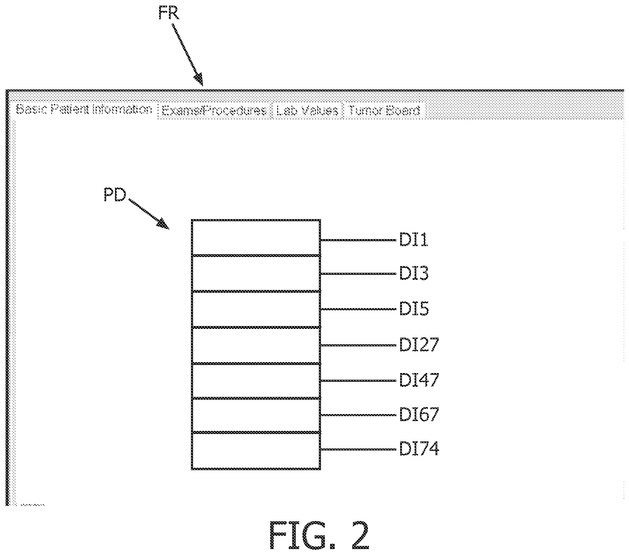

Fig. 2 illustrates the patient data structure PD comprising data items DI1, DI3, DI5, DI27, DI47, DI67, DI74, and a form FR for entering the data items. The form may be displayed on a display 120 and may facilitate the data entry by the user. In the context of oncology, for example, the data items will typically pertain to patient demographics and history, cancer characteristics such as tumor size or tumor stages, information about applied therapy, and results of diagnostic examinations. Data items may be grouped in a number of groups based on data types. For example, the first group may comprise basic patient information, the second group may comprise exams and procedures, the third group may comprise lab values and the fourth group may comprise tumor board data items. Each group may be displayed using tabs at the top of the form and entered by clicking on a tab corresponding to the group. A person skilled in the art will understand that there are many implementations of entering data items and that the scope of the claims is not limited to any particular implementation.

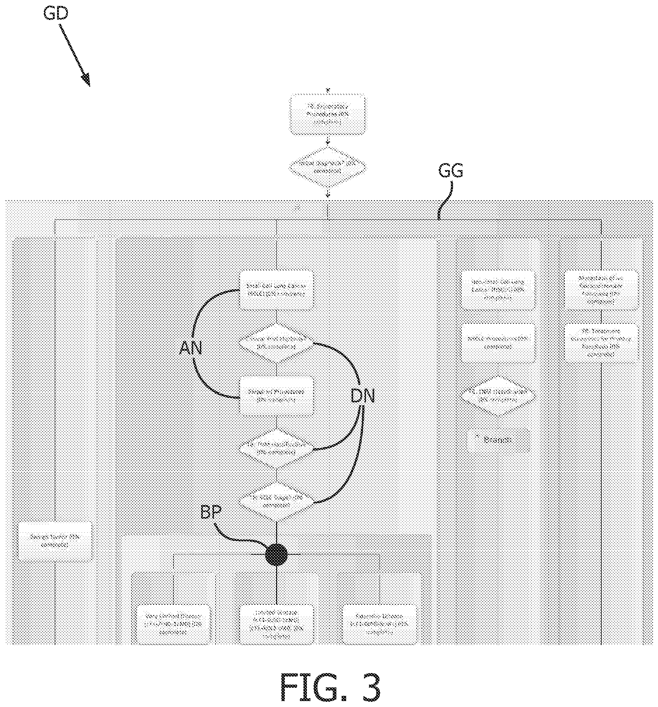

Fig. 3 illustrates the guideline data structure GD and the guideline graph GG. The guideline data structure GD may be a form or another object comprising a graph GG. Alternatively, the guideline data structure may be the guideline graph GG. The guideline graph is a directed graph, for example a tree. A node of the graph is said to be directly connected to another node if there is a directed edge from said node to said another node. A node is said to be indirectly connected to another node if said node is connected to a third node and the third node is directly connected to said another node. The guideline graph GG comprises action nodes AN and branching points BP. To facilitate interpretation of branching points BP, the graph may comprise decision nodes DN. The action nodes are associated with actions. Typical actions include, but are not limited to, lab tests, exams by physicians, and treatment procedures. The decision nodes comprise queries for describing the branching points BP.

The data items relating to patients are stored in a storage such as the memory 170. The system 100 comprises a linker 110 adapted for linking the data items stored in the storage to action nodes of the guideline graph. There is a relation between the data items comprised in the storage and the action nodes of the guideline graph: the data items are results of the actions associated with the action nodes. The linker 110 uses these relations: a data item is linked to an action node when it can be interpreted as a result of the action associated with the action node. For example, the blood pressure value (i.e. a data item) is a result of blood pressure measurement (i.e. an action). Other examples of this relation include, but are not limited to:
- body temperature and body temperature measurement;
- lack of pregnancy and pregnancy test;
- size of a nodule in the lungs and CAD analysis of a CT image of the thorax; and
- dose of radiation for treating a tumor and treatment of the tumor with radiation.

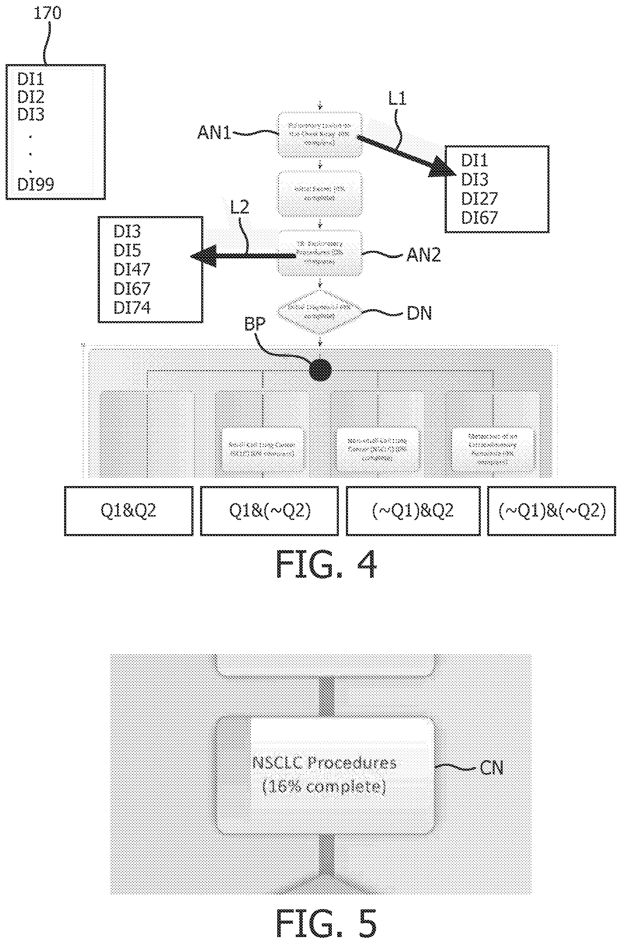

Fig. 4 schematically illustrates data items comprised in the data storage, linked to action nodes comprised in the guideline graph. Data items DI1, DI3, DI27 and DI67 comprised in the patient data structure PD shown in Fig. 2 are linked to node AN1. These data items are related to actions of the action node AN1. Similarly, data items, DI3, DI5, DI47, DI67 and DI74 comprised in the patient data structure PD shown in Fig. 2 are linked to node AN2. These data items are related to actions of the action node AN2.

The nodes, to which data items are linked, may be indicated on the display. A person skilled in the art will know various methods of indicating these nodes. For example, the text displayed in these nodes may be bolded or highlighted, the background and/or the border of the node may be colored, or the brightness of the node may oscillate within a certain range.

Fig. 4 further schematically illustrates a decision node DN. The decision node indicates a rule (typically at least one expression) to be computed for determining the next action node following the branching point BP. The rule is computed by a rule engine comprised in the guideline data structure, associated with the branching point. The rule is based on data items linked to action nodes connected to the branching point. For example, the rule may be based on four Boolean expressions, wherein only one may be true at a time: Q1 & Q2, Q1 & (∼Q2), (∼Q1) & Q2 and (∼Q1) & (∼Q2). Q1 and Q2 are logical expressions which evaluate to true or false depending on the data items DI1, DI3, DI5, DI27, DI47, DI67 and DI74 linked to action nodes AN1 and AN2 connected to the branching point BP.

Fig. 5 shows an exemplary completeness node CN for indicating a degree of completeness of an action comprised in an action node AN, connected directly or indirectly to the completeness node CN, computed by the completeness estimator 130. For example, the completeness estimator 130 may be arranged for computing the percentage of all data items linked to an action node directly connected to the completeness node, to all data items that need to be linked to said action node. The completeness may also relate to a procedure which combines actions associated with more than one action node connected to the completeness node CN. For example, the completeness estimator 130 may be arranged for computing the percentage of all data items linked to action nodes connected to the completeness node, which action nodes are associated with actions necessary for completion of a procedure. Alternatively, a completeness node can be included in an action node AN or a decision node DN. Such an action node AN or decision node DN will perform a function of the completeness node CN in addition to its AN- or DN-related node functions. The completeness may be shown as a number (e.g. a percentage or fraction) or as a progress bar, as illustrated in Fig. 5. A person skilled in the art will know other ways of showing the completeness.

The pathway finder 140 is adapted for computing a pathway of the patient in the guideline graph GG. The pathway comprises the at least one action node to which data items comprised in the patient data structure PD are linked. Thus, the pathway of the patient is determined by data items comprised in the patient data structure and the relation between said data items and actions associated with the action nodes comprised in the guideline graph. The pathway finder takes into account action nodes to which data items comprised in the patient data structure of a patient of interest are linked. Based on these action nodes, the pathway finder determines a possible pathway defined by the said action nodes and the guideline graph edges. At each branching point, the pathway finder is arranged for computing the branching rule and to select the branch according to the rule. If the patient data structure comprises most data items required by the medical guideline, it will be mapped into one pathway. However, in exceptional cases, or if the patient data is not complete, or if the patient data comprises data required by multiple medical procedures described in the guideline graph GG, the pathway may comprise more than one unconnected segment. Each segment may be related to a different medical procedure described in the guideline graph GG yielding the corresponding pathway. Entering more data items into the patient record may help to select the most appropriate pathway. Alternatively, the segments may describe the same pathway, one segment preceding the other. Entering more data items into the patient record may help to connect the segments to form one pathway. Optionally, the user may be allowed to correct the pathway. In addition or alternatively, the user interface may allow the user to select a segment of the pathway as the correct pathway.

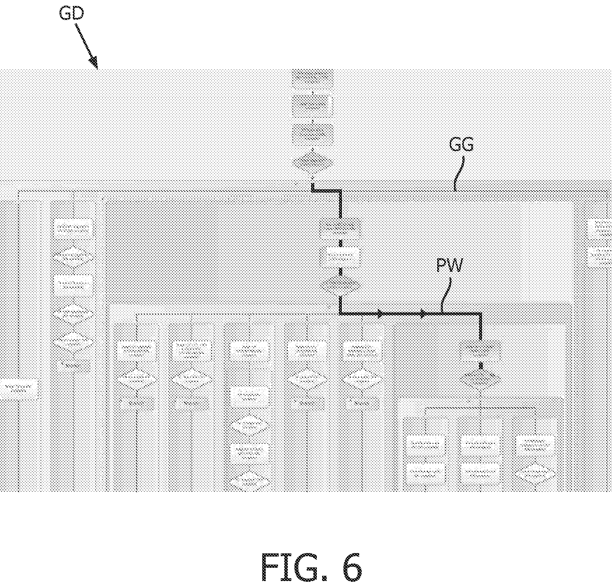

Fig. 6 shows an exemplary pathway PW in the guideline graph GG comprised in the guideline data structure GD. The pathway PW is indicated by drawing edges with a bolder and darker line. Optionally, colors can be used. Alternatively or in addition, the pathway may be indicated by indicating the nodes of the pathway.

The predictor 150 is arranged for selecting, on the basis of the pathway (PW) and the guideline graph (GG), at least one action node of the guideline graph (GG), which is not yet comprised in the pathway (PW), and for including the selected at least one action node in the pathway (PW). The new action node may be a node extending the pathway beyond the last action node of the pathway or may be a missing action node for joining two segments of the pathway. The action associated with the new node may suggest treatment to be applied to or tests/measurements to be performed on the patient. Thus, the system of the invention may be a valuable tool for implementing decision support systems.

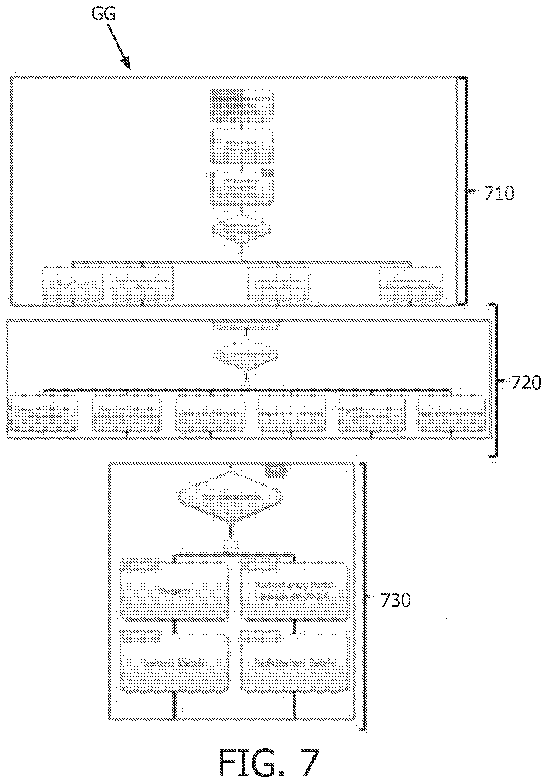

In many clinical situations, it is important to be able to check if all necessary therapeutic or diagnostics steps comprised in a care phase of a complete care cycle have been performed before the next step can be made. In the context of oncology treatment, the care phases may include, for example, diagnosis, staging, therapy, and adjuvant therapy. Decisions are usually made by means of multidisciplinary tumor conferences. It is important that all needed information is available before discussing a patient at the conference. Missing information or missing diagnostics steps discovered during the meeting often leads to a rescheduling of the patient for the next tumor conference and thus, to a delay in the process. To assist multidisciplinary tumor conference participants, in an embodiment of the system 100, a care phase is assigned to nodes comprised in the guideline graph. Optionally, a label of the assigned care phase may be displayed with each node of the guideline graph. Alternatively, the system 100 may comprise means for assigning a care phase to each node of a plurality of nodes of the guideline, preferably to each action node of the guideline. Fig. 7 shows a guideline wherein a care phase is assigned to each node: 710 - diagnostic care phase, 720 - staging care phase, and 730 - therapy care phase.

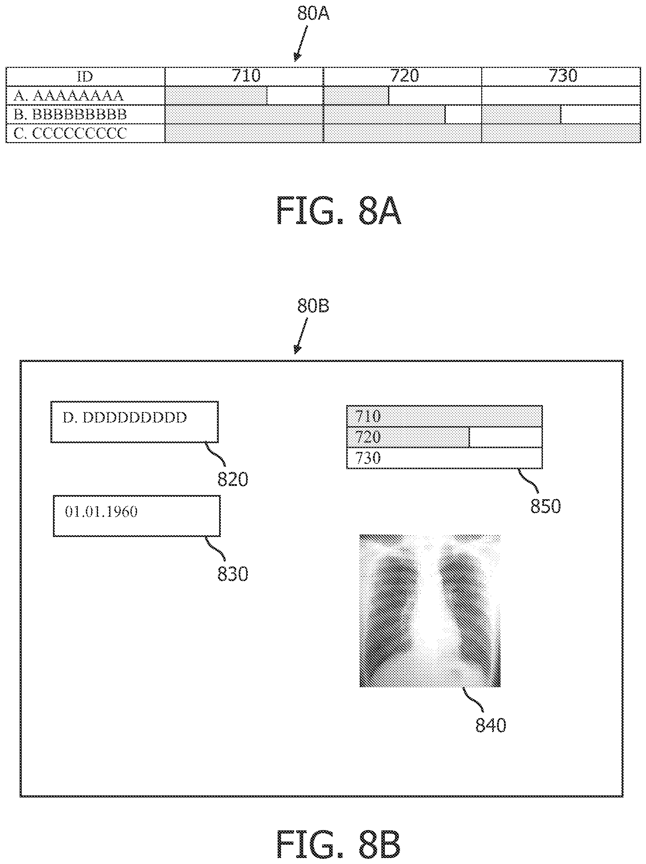

In an embodiment, the system 100 further comprises a care phase completeness estimator for computing the degree of completeness of the care phase based on the computed degree of completeness of the action comprised in each action node corresponding to the care phase. The degree of completeness of the care phase may be the mean or weighted mean of the degrees of completeness of actions comprised in action nodes corresponding to the care phase, wherein the weights may rank the actions. Figs. 8A and 8B illustrate how to visualize care phase completeness on a list of patient summaries 80A and on a patient data form 80B, respectively. In Fig. 8A, the first column of the list displays identification data ID of each patient. The data may comprise patient's name (A. AAAAAAAA. B. BBBBBBBB, and C. CCCCCCCC), sex, date of birth, age, etc. In the second, third and fourth columns the degrees of completeness care phases 710, 720 and 730, respectively, are indicated for each patient. In Fig. 8B, the exemplary patient form 80B comprises patient name field 820 with the name D. DDDDDD, date of birth field with the date of 01.01.1960, patient's chest x-ray image 850 and exemplary indicators of the degree of completeness of each care phase of the three care phases 710, 720 and 730. A person skilled in the art will be able to add various graphical representations indicating the degrees of completeness of various care phases of various patients to various lists of patient summaries and various patient data forms.

A person skilled in the art will appreciate that the system 100 may be a valuable tool for assisting a physician in many aspects of her/his job. Further, although the embodiments of the system are illustrated using medical applications of the system, non-medical applications of the system are also contemplated.

Those skilled in the art will further understand that other embodiments of the system 100 are also possible. It is possible, among other things, to redefine the units of the system and to redistribute their functions. Although the described embodiments apply to medical images, other applications of the system, not related to medical applications, are also possible.

The units of the system 100 may be implemented using a processor. Normally, their functions are performed under the control of a software program product. During execution, the software program product is normally loaded into a memory, like a RAM, and executed from there. The program may be loaded from a background memory, such as a ROM, hard disk, or magnetic and/or optical storage, or may be loaded via a network like the Internet. Optionally, an application-specific integrated circuit may provide the described functionality.

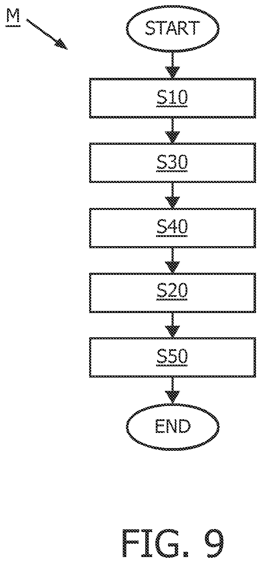

Fig. 9 shows an exemplary flowchart of the method M of mapping a patient data structure PD for describing a patient's case into a guideline data structure GD for describing a medical guideline, wherein the patient data structure PD comprises data items of a plurality of data items DI1, DI2, ..., DI99, the guideline data structure GD comprises a guideline graph GG, wherein the guideline graph GG is a directed graph, the guideline graph GG comprising action nodes AN1, AN2, wherein each action node AN1, AN2 is associated with an action. The method begins with linking S10 data items DI1, DI3, DI5, DI27, DI47, DI67, DI74 comprised in the patient data structure PD to action nodes AN1, AN2 of the guideline graph GG, based on a relation between said data items and actions associated with said action nodes, thereby mapping the patient data structure PD into the guideline data structure GD. After said linking S10, the method M continues to computing S30 a degree of completeness of actions comprised in some action nodes. After said computing S30, the method M continues to finding S40 a pathway PW of the patient in the guideline graph GG, the pathway PW comprising the at least one action node AN1, AN2 to which data items comprised in the patient data structure PD are linked. After said finding S40, the method continues to displaying S20 at least part of the guideline graph GG, wherein said at least part of the guideline graph comprises at least one action node AN1, AN2 to which data items comprised in the patient data structure PD are linked, and wherein said at least one action node AN1, AN2 is indicated in the displayed part of the guideline graph GG. Optionally, after said displaying S20, the method M continues to selecting S50, on the basis of the pathway PW and the guideline graph GG, at least one action node of the guideline graph GG which is not yet comprised in the pathway PW, which selected at least one action node is included in the pathway PW. After said selecting S50, the method terminates.

A person skilled in the art may change the order of some steps, add some optional steps (e.g. user interaction for manually selecting action nodes in the guideline graph to be added to a pathway displayed on the display) or omit some non-mandatory steps, or perform some steps concurrently using threading models, multi-processor systems or multiple processes without departing from the concept as intended by the present invention. Optionally, two or more steps of the method M may be combined into one step. Optionally, a step of the method M may be split into a plurality of steps.

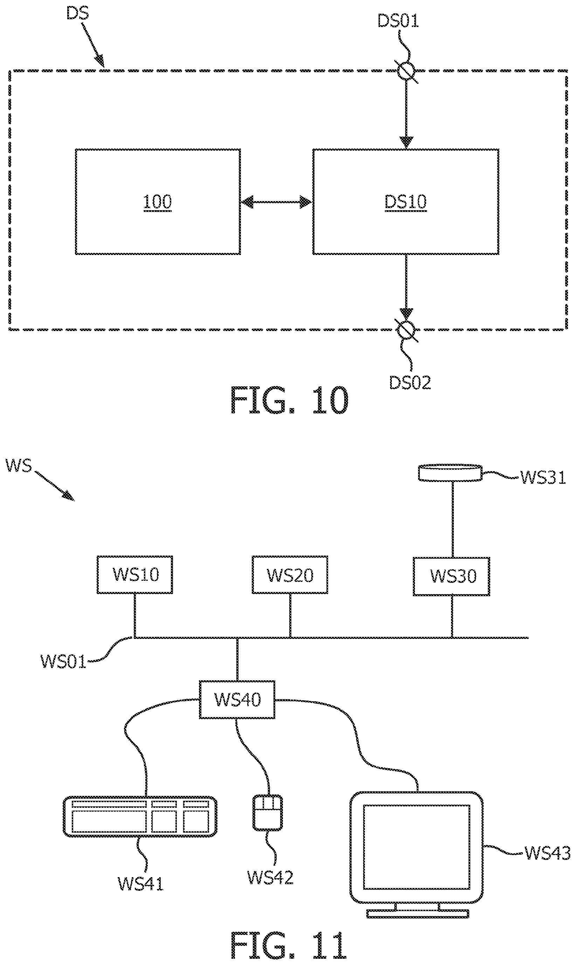

Fig. 10 schematically shows an exemplary embodiment of a decision support system DS employing the system 100 of the invention, said decision support system DS comprising a decision support unit DS10 connected via an internal connection with the system 100, an input connector DS01, and an output connector DS02. This arrangement advantageously increases the capabilities of the decision support system DS, providing said decision support system DS with advantageous capabilities of the system 100.

Fig. 11 schematically shows an exemplary embodiment of the workstation WS. The workstation comprises a system bus WS01. A processor WS10, a memory WS20, a disk input/output (I/O) adapter WS30, and a user interface (UI) WS40 are operatively connected to the system bus WS01. A disk storage device WS31 is operatively coupled to the disk I/O adapter WS30. A keyboard WS41, a mouse WS42, and a display WS43 are operatively coupled to the UI WS40. The system 100 of the invention, implemented as a computer program, is stored in the disk storage device WS31. The workstation WS00 is arranged to load the program and input data into memory WS20 and execute the program on the processor WS10. The user can input information to the workstation WS00, using the keyboard WS41 and/or the mouse WS42. The workstation is arranged to output information to the display device WS43 and/or to the disk WS31. A person skilled in the art will understand that there are numerous other embodiments of the workstation WS known in the art and that the present embodiment serves the purpose of illustrating the invention and must not be interpreted as limiting the invention to this particular embodiment.

It should be noted that the above-mentioned embodiments illustrate rather than limit the invention and that those skilled in the art will be able to design alternative embodiments without departing from the scope of the appended claims. In the claims, any reference signs placed between parentheses shall not be construed as limiting the claim. The word "comprising" does not exclude the presence of elements or steps not listed in a claim or in the description. The word "a" or "an" preceding an element does not exclude the presence of a plurality of such elements. The invention can be implemented by means of hardware comprising several distinct elements and by means of a programmed computer. In the system claims enumerating several units, several of these units can be embodied by one and the same record of hardware or software. The usage of the words first, second, third, etc., does not indicate any ordering. These words are to be interpreted as names.

## Claims

1. A system (100) for mapping a patient data structure (PD) for describing a patient's case into a guideline data structure (GD) for describing a medical guideline, the system comprising a user interface (165) for receiving user input comprising data items (DI1; DI3; DI5; DI27; DI47, DI67, DI74) of a plurality of data items (DI1; DI2; ...; DI99) for the patient data structure (PD) and storage (170) for storing:
- the plurality of data items (DI1; DI2; ...; DI99);
- the patient data structure (PD) comprising the data items (DI1; DI3; DI5; DI27; DI47, DI67, DI74) of the plurality of data items (DI1; DI2; ...; DI99);
- the guideline data structure (GD) comprising a guideline graph (GG), wherein the guideline graph (GG) is a directed graph, the guideline graph (GG) comprising action nodes (AN1; AN2), wherein each action node (AN1; AN2) is associated with an action, wherein an action associated with each action node comprises to perform a measurement on and/or apply a treatment to the patient;
- the system further comprising a linker (110) for linking data items (DI1; DI3; DI5; DI27; DI47, DI67, DI74) comprised in the patient data structure (PD) to action nodes (AN1; AN2) of the guideline graph (GG), based on a relation between said data items and actions associated with said action nodes (AN1; AN2), thereby mapping the patient data structure (PD) into the guideline data structure (GD), wherein the relation between the data items and actions comprises a measurement outcome for the action to perform a measurement and/or parameters of the applied treatment for the action to apply a treatment to the patient; wherein the system is adapted such that the receiving of the user input comprising the data items (DI1; DI3; DI5; DI27; DI47, DI67, DI74) of the patient data structure and the guideline graph are decoupled and such that the user input, which comprises the data items (DI1; DI3; DI5; DI27; DI47, DI67, DI74) of the patient data structure, is receivable out of an order of the action nodes defined by the guideline graph;
- the system further comprising a pathway finder (140) for computing a pathway (PW) of the patient in the guideline graph (GG), the pathway comprising the action nodes (AN1; AN2) to which data items comprised in the patient data structure are linked, and wherein the pathway is determined by data items comprised in the patient data structure and the relation between said data items and actions associated with the action nodes comprised in the guideline graph, wherein the pathway provides an overview of a personalized medical guideline showing actions which have already been completed and which need to be completed.

2. A system (100) as claimed in claim 1, wherein the guideline graph (GG) further comprises decision nodes (DN), and wherein each decision node (DN) is adapted for testing data items linked to action nodes (AN1; AN2) connected directly or indirectly to said decision node (DN).

3. A system (100) as claimed in claim 1, further comprising a display (120) for displaying at least part of the guideline graph (GG), wherein said at least part of the guideline graph comprises at least one action node (AN1; AN2) to which data items comprised in the patient data structure (PD) are linked, and wherein said at least one action node (AN1; AN2) is indicated in the displayed part of the guideline graph (GG).

4. A system (100) as claimed in claim 1, further comprising a completeness estimator (130) for computing a degree of completeness of an action comprised in an action node.

5. A system (100) as claimed in claim 4, wherein the guideline graph (GG) further comprises a completeness node (CN) for indicating the computed degree of completeness.

6. A system (100) as claimed in claim 1, further comprising a predictor (150) for selecting, on the basis of the pathway (PW) and the guideline graph (GG), at least one action node of the guideline graph (GG) which is not yet comprised in the pathway (PW), and for including the selected at least one action node in the pathway (PW).

7. A system (100) as claimed in any one of the previous claims, wherein a care phase is assigned to nodes in the guideline graph (GG).

8. A system (100) as claimed in claim 4, wherein a care phase is assigned to nodes in the guideline graph (GG) and at least one of said nodes is an action node, the system further comprising a care phase completeness estimator for computing the degree of completeness of the care phase based on the computed degree of completeness of the action comprised in the action node.

9. A decision support system (DS) comprising a system (100) as claimed in any one of the previous claims.

10. A workstation (WS) comprising a system (100) as claimed in any one of the previous claims.

11. A computer-implemented method (M) of mapping a patient data structure (PD) for describing a patient's case into a guideline data structure (GD) for describing a medical guideline, wherein:
- the method comprising receiving user input comprising data items (DI1; DI3; DI5; DI27; DI47, DI67, DI74) of a plurality of data items (DI1; DI2; ...; DI99) for the patient data structure (PD);
- the patient data structure (PD) comprises the data items (DI1; DI3; DI5; DI27; DI47, DI67, DI74) of the plurality of data items (DI1; DI2; ...; DI99);
- the guideline data structure (GD) comprises a guideline graph (GG), wherein the guideline graph (GG) is a directed graph, the guideline graph (GG) comprising action nodes (AN1; AN2), wherein each action node (AN1; AN2) is associated with an action, wherein an action associated with each action node comprises to perform a measurement on and/or apply a treatment to the patient;
- the method comprising linking (S10) data items (DI1; DI3; DI5; DI27; DI47, DI67, DI74) comprised in the patient data structure (PD) to action nodes (AN1; AN2) of the guideline graph (GG), based on a relation between said data items and actions associated with said action nodes, thereby mapping the patient data structure (PD) into the guideline data structure (GD), wherein the relation between the data items and actions comprises a measurement outcome for the action to perform a measurement and/or parameters of the applied treatment for the action to apply a treatment to the patient; wherein the receiving of the user input comprising the data items (DI1; DI3; DI5; DI27; DI47, DI67, DI74) of the patient data structure and the guideline graph are decoupled and the user input, which comprises the data items (DI1; DI3; DI5; DI27; DI47, DI67, DI74) of the patient data structure, is receivable out of an order of the action nodes defined by the guideline graph; and
- the method comprising computing a pathway (PW) of the patient in the guideline graph (GG), the pathway comprising the action nodes (AN1; AN2) to which data items comprised in the patient data structure (PD) are linked, and wherein the pathway is determined by data items comprised in the patient data structure and the relation between said data items and actions associated with the action nodes comprised in the guideline graph, wherein the pathway provides an overview of a personalized medical guideline showing actions which have already been completed and which need to be completed.

12. A computer program product to be loaded by a computer arrangement, comprising instructions for mapping a patient data structure for describing a patient's case into a guideline data structure for describing a medical guideline, the computer arrangement comprising a processing unit and a memory, the computer program product, after being loaded, providing said processing unit with the capability to carry out steps of a method as claimed in claim 11.

## Patentansprüche

1. System (100) zum Abbilden einer Patientendatenstruktur (PD) zum Beschreiben des Falls eines Patienten in einer Richtliniendatenstruktur (GD) zum Beschreiben einer medizinischen Richtlinie, wobei das System umfasst: eine Benutzerschnittstelle (165) zum Empfangen von Benutzereingabe, umfassend Datenelemente (DI1; DI3; DI5; DI27; DI47, DI67, DI74) aus einer Vielzahl von Datenelementen (DI1; DI2; ...; DI99) für die Patientendatenstruktur (PD) und Speicher (170) zum Speichern:
- der Vielzahl von Datenelementen (DI1; DI2; ...; DI99);
- der Patientendatenstruktur (PD), umfassend die Datenelemente (DI1; DI3; DI5; DI27; DI47, DI67, DI74) der Vielzahl von Datenelementen (DI1; DI2; ...; DI99);
- der Richtliniendatenstruktur (GD), umfassend ein Richtliniendiagramm (GG), wobei das Richtliniendiagramm (GG) ein geführtes Diagramm ist, wobei das Richtliniendiagramm (GG) Aktionsknoten (AN1; AN2) umfasst, wobei jeder Aktionsknoten (AN1; AN2) mit einer Aktion verbunden ist, wobei eine mit jedem Aktionsknoten verbundene Aktion das Ausführen einer Messung und/oder Abwenden einer Behandlung auf den Patienten umfasst;
- wobei das System weiter einen Verknüpfer (110) zum Verknüpfen von Datenelementen (DI1; DI3; DI5; DI27; DI47, DI67, DI74), welche in der Patientendatenstruktur (PD) enthalten sind, mit Aktionsknoten (AN1; AN2) des Richtliniendiagramms (GG) umfasst, basierend auf einer Beziehung zwischen den Datenelementen und Aktionen, welche mit den Aktionsknoten (AN1; AN2) verbunden sind, wodurch die Patientendatenstruktur (PD) in der Richtliniendatenstruktur (GD) abgebildet wird, wobei die Beziehung zwischen den Datenelementen und Aktionen ein Messergebnis zum Durchführen einer Messung durch die Aktion und/oder Parameter der angewendeten Behandlung zum Anwenden einer Behandlung auf den Patienten durch die Aktion umfasst; wobei das System adaptiert ist, sodass das Empfangen der Benutzereingabe, umfassend die Datenelemente (DI1; DI3; DI5; DI27; DI47, DI67, DI74) der Patientendatenstruktur und des Richtliniendiagramms entkoppelt sind, und sodass die Benutzereingabe, umfassend die Datenelemente (DI1; DI3; DI5; DI27; DI47, DI67, DI74) der Patientendatenstruktur aus einer Anforderung der durch das Richtliniendiagramm definierten Aktionsknoten empfangbar ist;
- wobei das System weiter einen Pfadfinder (140) zum Berechnen eines Pfads (PW) des Patienten in dem Richtliniendiagramm (GG) umfasst, wobei der Pfad die Aktionsknoten (AN1; AN2) umfasst, mit welchen in der Patientendatenstruktur enthaltene Datenelemente verknüpft sind, und wobei der Pfad durch in der Patientendatenstruktur enthaltene Datenelemente und die Beziehung zwischen den Datenelementen und Aktionen, welche mit den in der Patientendatenstruktur enthaltenen Aktionsknoten verbunden sind, bestimmt ist, wobei der Pfad einen Überblick über eine personalisierte medizinische Richtlinie bereitstellt, welche Aktionen zeigt, welche bereits abgeschlossen wurden und welche abgeschlossen werden müssen.

2. System (100) nach Anspruch 1, wobei das Richtliniendiagramm (GG) weiter Entscheidungsknoten (DN) umfasst, und wobei jeder Entscheidungsknoten (DN) für das Überprüfen von mit Aktionsknoten (AN1; AN2) verknüpften Datenelementen adaptiert ist, welche direkt oder indirekt mit dem Entscheidungsknoten (DN) verknüpft sind.

3. System (100) nach Anspruch 1, weiter eine Anzeige (120) zum Anzeigen zumindest eines Teils des Richtliniendiagramms (GG) umfassend, wobei der zumindest Teil des Richtliniendiagramms zumindest einen Aktionsknoten (AN1; AN2) umfasst, mit welchem in der Patientendatenstruktur (PD) enthaltene Datenelemente verknüpft sind, und wobei der zumindest eine Aktionsknoten (AN1; AN2) in dem angezeigten Teil des Richtliniendiagramms (GG) angegeben ist.

4. System (100) nach Anspruch 1, weiter einen Vollständigkeitsschätzer (130) zum Berechnen eines Grads der Vollständigkeit einer in einem Aktionsknoten enthaltenen Aktion umfassend.

5. System (100) nach Anspruch 4, wobei das Richtliniendiagramm (GG) weiter einen Vollständigkeitsknoten (CN) zum Angeben des berechneten Grads der Vollständigkeit umfasst.

6. System (100) nach Anspruch 1, weiter einen Vorhersager (150) zum Auswählen, auf der Basis des Pfades (PW) und des Richtliniendiagramms (GG), zumindest eines Aktionsknotens des Richtliniendiagramms (GG), welcher noch nicht in dem Pfad (PW) enthalten ist, und zum Einschließen des ausgewählten zumindest einen Aktionsknotens in den Pfad (PW) umfassend.

7. System (100) nach einem der vorstehenden Ansprüche, wobei Knoten in dem Richtliniendiagramm (GG) eine Pflegephase zugewiesen wird.

8. System (100) nach Anspruch 4, wobei Knoten in dem Richtliniendiagramm (GG) eine Pflegephase zugewiesen wird und zumindest einer von den Knoten ein Aktionsknoten ist, wobei das System weiter einen Pflegephasenvollständigkeitsschätzer zum Berechnen des Grads der Vollständigkeit der Pflegephase auf Basis des berechneten Grads der Vollständigkeit der in dem Aktionsknoten enthaltenen Aktion umfasst.

9. Entscheidungsunterstützungssystem (DS), umfassend ein System (100) nach einem der vorstehenden Ansprüche.

10. Arbeitsstation (WS), umfassend ein System (100) nach einem der vorstehenden Ansprüche.

11. Computerimplementiertes Verfahren (M) des Abbildens einer Patientendatenstruktur (PD) zum Beschreiben des Falls eines Patienten in einer Richtliniendatenstruktur (GD) zum Beschreiben einer medizinischen Richtlinie, wobei:
- das Verfahren Empfangen von Benutzereingabe, umfassend Datenelemente (DI1; DI3; DI5; DI27; DI47, DI67, DI74) aus einer Vielzahl von Datenelementen (DI1; DI2; ...; DI99) für die Patientendatenstruktur (PD) umfasst;
- der Patientendatenstruktur (PD) die Datenelemente (DI1; DI3; DI5; DI27; DI47, DI67, DI74) der Vielzahl von Datenelementen (DI1; DI2; ...; DI99) umfasst;
- die Richtliniendatenstruktur (GD) ein Richtliniendiagramm (GG) umfasst, wobei das Richtliniendiagramm (GG) ein geführtes Diagramm ist, wobei das Richtliniendiagramm (GG) Aktionsknoten (AN1; AN2) umfasst, wobei jeder Aktionsknoten (AN1; AN2) mit einer Aktion verbunden ist, wobei eine mit jedem Aktionsknoten verbundene Aktion das Ausführen einer Messung und/oder Abwenden einer Behandlung auf den Patienten umfasst;
- das Verfahren Verknüpfen (S10) von Datenelementen (DI1; DI3; DI5; DI27; DI47, DI67, DI74), welche in der Patientendatenstruktur (PD) enthalten sind, mit Aktionsknoten (AN1; AN2) des Richtliniendiagramms (GG) umfasst, basierend auf einer Beziehung zwischen den Datenelementen und Aktionen, welche mit den Aktionsknoten verbunden sind, wodurch die Patientendatenstruktur (PD) in der Richtliniendatenstruktur (GD) abgebildet wird, wobei die Beziehung zwischen den Datenelementen und Aktionen ein Messergebnis zum Durchführen einer Messung durch die Aktion und/oder Parameter der angewendeten Behandlung zum Anwenden einer Behandlung auf den Patienten durch die Aktion umfasst; wobei das Empfangen der Benutzereingabe, umfassend die Datenelemente (DI1; DI3; DI5; DI27; DI47, DI67, DI74) der Patientendatenstruktur und des Richtliniendiagramms entkoppelt sind und die Benutzereingabe, umfassend die Datenelemente (DI1; DI3; DI5; DI27; DI47, DI67, DI74) der Patientendatenstruktur aus einer Anforderung der durch das Richtliniendiagramm definierten Aktionsknoten empfangbar ist; und
- das Verfahren Berechnen eines Pfads (PW) des Patienten in dem Richtliniendiagramm (GG) umfasst, wobei der Pfad die Aktionsknoten (AN1; AN2) umfasst, mit welchen in der Patientendatenstruktur (PD) enthaltene Datenelemente verknüpft sind, und wobei der Pfad durch in der Patientendatenstruktur enthaltene Datenelemente und die Beziehung zwischen den Datenelementen und Aktionen, welche mit den in der Patientendatenstruktur enthaltenen Aktionsknoten verbunden sind, bestimmt ist, wobei der Pfad einen Überblick über eine personalisierte medizinische Richtlinie bereitstellt, welche Aktionen zeigt, welche bereits abgeschlossen wurden und welche abgeschlossen werden müssen.

12. Computerprogrammprodukt, welches von einer Computeranordnung geladen werden soll, umfassend Anweisungen zum Abbilden einer Patientendatenstruktur zum Beschreiben des Falls eines Patienten in einer Richtliniendatenstruktur zum Beschreiben einer medizinischen Richtlinie, wobei die Computeranordnung eine Verarbeitungseinheit und einen Speicher umfasst, wobei das Computerprogrammprodukt, nachdem es geladen wurde, für die Verarbeitungseinheit die Möglichkeit bereitstellt, Schritte eines Verfahrens nach Anspruch 11 auszuführen.

## Revendications

1. Système (100) de mappage d'une structure de données de patient (PD) pour la description d'un cas de patient dans une structure de données de lignes directrices (GD) pour la description d'une ligne directrice médicale, le système comprenant une interface utilisateur (165) pour la réception d'une entrée d'utilisateur comprenant des éléments de données (DI1 ; DI3 ; DI5 ; DI27 ; DI47 ; DI67 ; DI74) d'une pluralité d'éléments de données (DI1 ; DI2 ; ... ; DI99) pour la structure de données de patient (PD) et le stockage (170) pour stocker :
- la pluralité d'éléments de données (DI1 ; DI2 ; ... ; DI99) ;
- la structure de données de patient (PD) comprenant les éléments de données (DI1 ; DI3 ; DI5 ; DI27 ; DI47 ; DI67 ; DI74) de la pluralité d'éléments de données (DI1 ; DI2 ; ... ; DI99) ;
- la structure de données de lignes directrices (GD) comprenant un graphique de lignes directrices (GG), dans lequel le graphique de lignes directrices (GG) est un graphique dirigé, le graphique de lignes directrices (GG) comprenant des noeuds d'action (AN1 ; AN2), dans lequel chaque noeud d'action (AN1 ; AN2) est associé à une action, dans lequel une action associée à chaque noeud d'action comprend la réalisation d'une mesure sur et/ou l'application d'un traitement au patient ;
- le système comprenant en outre un lieur (110) pour lier des éléments de données (DI1 ; DI3 ; DI5 ; DI27 ; DI47 ; DI67 ; DI74) compris dans la structure de données de patient (PD) à des noeuds d'action (AN1 ; AN2) du graphique de lignes directrices (GG), sur la base d'une relation entre lesdits éléments de données et actions associées auxdits noeuds d'action (AN1 ; AN2), mappant ce faisant la structure de données de patient (PD) dans la structure de données de lignes directrices (GD), dans lequel la relation entre les éléments de données et actions comprend un résultat de mesure pour l'action de réaliser une mesure et/ou des paramètres du traitement appliqué pour l'action d'appliquer un traitement au patient ; dans lequel le système est adapté de sorte que la réception de l'entrée d'utilisateur comprenant les éléments de données (DI1 ; DI3 ; DI5 ; DI27 ; DI47 ; DI67 ; DI74) de la structure de données de patient et le graphique de lignes directrices sont découplés et tels que l'entrée d'utilisateur, qui comprend les éléments de données (DI1 ; DI3 ; DI5 ; DI27 ; DI47 ; DI67 ; DI74) de la structure de données de patient, est recevable hors d'un ordre des noeuds d'action définis par le graphique de lignes directrices ;
- le système comprenant en outre un découvreur de voie (140) pour le calcul d'une voie (PW) du patient dans le graphique de lignes directrices (GG), la voie comprenant les noeuds d'action (AN1 ; AN2) auxquels des éléments de données compris dans la structure de données de patient sont liés, et dans lequel la voie est déterminée par des éléments de données compris dans la structure de données de patient et la relation entre lesdits éléments de données et actions associées aux noeuds d'action compris dans le graphique de lignes directrices, dans lequel la voie fournit un aperçu d'une ligne directrice médicale personnalisée montrant des actions qui sont déjà terminées et qui ont besoin d'être terminées.

2. Système (100) selon la revendication 1, dans lequel le graphique de lignes directrices (GG) comprend en outre des noeuds de décision (DN), et dans lequel chaque noeud de décision (DN) est adapté pour tester des éléments de données liés à des noeuds d'action (AN1 ; AN2) reliés directement ou indirectement audit noeud de décision (DN).

3. Système (100) selon la revendication 1, comprenant en outre un écran (120) pour l'affichage d'au moins une partie du graphique de lignes directrices (GG), dans lequel ladite au moins une partie du graphique de lignes directrices comprend au moins un noeud d'action (AN1 ; AN2) auquel des éléments de données compris dans la structure de données de patient (PD) sont liés, et dans lequel ledit au moins un noeud d'action (AN1 ; AN2) est indiqué dans la partie affichée du graphique de lignes directrices (GG).

4. Système (100) selon la revendication 1, comprenant en outre un estimateur de complétude (130) pour calculer un degré de complétude d'une action comprise dans un noeud d'action.

5. Système (100) selon la revendication 4, dans lequel le graphique de lignes directrices (GG) comprend en outre un noeud de complétude (CN) pour indiquer le degré de complétude calculé.

6. Système (100) selon la revendication 1, comprenant en outre un prédicteur (150) pour sélectionner, sur la base de la voie (PW) et du graphique de lignes directrices (GG), au moins un noeud d'action du graphique de lignes directrices (GG) qui n'est pas encore compris dans la voie (PW), et pour inclure l'au moins un noeud d'action sélectionné dans la voie (PW).

7. Système (100) selon l'une quelconque des revendications précédentes, dans lequel une phase de soin est attribuée à des noeuds dans le graphique de lignes directrices (GG).

8. Système (100) selon la revendication 4, dans lequel une phase de soin est attribuée à des noeuds dans le graphique de lignes directrices (GG) et au moins un desdits noeuds est un noeud d'action, le système comprenant en outre un estimateur de complétude de phase de soin pour le calcul du degré de complétude de la phase de soin sur la base du degré de complétude calculé de l'action comprise dans le noeud d'action.

9. Système de support de décision (DS) comprenant un système (100) selon l'une quelconque des revendications précédentes.

10. Poste de travail (WS) comprenant un système (100) selon l'une quelconque des revendications précédentes.

11. Procédé mis en oeuvre par ordinateur (M) de mappage d'une structure de données de patient (PD) pour la description d'un cas de patient dans une structure de données de lignes directrices (GD) pour la description d'une ligne directrice médicale, dans lequel :
- le procédé comprenant la réception d'une entrée d'utilisateur comprenant des éléments de données (DI1 ; DI3 ; DI5 ; DI27 ; DI47 ; DI67 ; DI74) d'une pluralité d'éléments de données (DI1 ; DI2 ; ... ; DI99) pour la structure de données de patient (PD) ;
- la structure de données de patient (PD) comprend les éléments de données (DI1 ; DI3 ; DI5 ; DI27 ; DI47 ; DI67 ; DI74) de la pluralité d'éléments de données (DI1 ; DI2 ; ... ; DI99) ;
- la structure de données de lignes directrices (GD) comprend un graphique de lignes directrices (GG), dans lequel le graphique de lignes directrices (GG) est un graphique dirigé, le graphique de lignes directrices (GG) comprenant des noeuds d'action (AN1 ; AN2), dans lequel chaque noeud d'action (AN1 ; AN2) est associé à une action, dans lequel une action associée à chaque noeud d'action comprend la réalisation d'une mesure sur et/ou l'application d'un traitement au patient ;
- le procédé comprenant en outre la liaison (S10) d'éléments de données (DI1 ; DI3 ; DI5 ; DI27 ; DI47 ; DI67 ; DI74) compris dans la structure de données de patient (PD) à des noeuds d'action (AN1 ; AN2) du graphique de lignes directrices (GG), sur la base d'une relation entre lesdits éléments de données et actions associées auxdits noeuds d'action, mappant ce faisant la structure de données de patient (PD) dans la structure de données de lignes directrices (GD), dans lequel la relation entre les éléments de données et actions comprend un résultat de mesure pour l'action de réaliser une mesure et/ou des paramètres du traitement appliqué pour l'action d'appliquer un traitement au patient ; dans lequel la réception de l'entrée d'utilisateur comprenant les éléments de données (DI1 ; DI3 ; DI5 ; DI27 ; DI47 ; DI67 ; DI74) de la structure de données de patient et le graphique de lignes directrices sont découplés et l'entrée d'utilisateur, qui comprend les éléments de données (DI1 ; DI3 ; DI5 ; DI27 ; DI47 ; DI67 ; DI74) de la structure de données de patient, est recevable hors d'un ordre des noeuds d'action définis par le graphique de lignes directrices ; et
- le procédé comprenant le calcul d'une voie (PW) du patient dans le graphique de lignes directrices (GG), la voie comprenant les noeuds d'action (AN1 ; AN2) auxquels des éléments de données compris dans la structure de données de patient (PD) sont liés, et dans lequel la voie est déterminée par des éléments de données compris dans la structure de données de patient et la relation entre lesdits éléments de données et actions associées aux noeuds d'action compris dans le graphique de lignes directrices, dans lequel la voie fournit un aperçu d'une ligne directrice médicale personnalisée montrant des actions qui sont déjà terminées et qui ont besoin d'être terminées.

12. Produit de programme informatique à charger par un agencement informatique, comprenant des instructions de mappage d'une structure de données de patient pour la description d'un cas de patient dans une structure de données de lignes directrices pour la description d'une ligne directrice médicale, l' agencement informatique comprenant une unité de traitement et une mémoire, le produit de programme informatique, après avoir été chargé, fournissant à ladite unité de traitement la capacité d'effectuer des étapes d'un procédé selon la revendication 11.
